(19) Europäisches Patentamt
European Patent Office
Office européen des brevets

(11) Veröffentlichungsnummer: **0 073 514**
**B1**

(12) **EUROPÄISCHE PATENTSCHRIFT**

(45) Veröffentlichungstag der Patentschrift: **31.07.85**

(51) Int. Cl.⁴: **G 01 N 33/53**, G 01 N 33/70, G 01 N 33/531

(21) Anmeldenummer: **82107973.8**

(22) Anmeldetag: **30.08.82**

(54) **Creatinin-Antikörper.**

(30) Priorität: **02.09.81 DE 3134787**

(43) Veröffentlichungstag der Anmeldung: **09.03.83 Patentblatt 83/10**

(45) Bekanntmachung des Hinweises auf die Patenterteilung: **31.07.85 Patentblatt 85/31**

(84) Benannte Vertragsstaaten: **AT BE CH DE FR GB IT LI LU NL SE**

(56) Entgegenhaltungen:
**EP-A-0 010 405**
**DE-A-2 811 537**

**CHEMICAL AND PHARMACEUTICAL BULLETIN, Band 28, Nr. 12, 1980, Tokio, T. KINOSHITA et al. "A *fluorophotometric determination of serum creatinine* and creatine using a creatinineamidohydrolase-creatineamidinohydrolase-sarcosine oxidase-peroxidase sytem and *diacetyldichlorofluorescin*", Seiten 3501 bis 3506**
**CLINICAL CHEMISTRY, Band 26, Nr. 8, Juli 1980, S. NARAYANAN et al. "Creatinine: A review", Seiten 1119 - 1126**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Neumann, Ulrich, Dr., Drosselweg 2, D-8123 Peissenberg (DE)**
Erfinder: **Ziegenhorn, Joachim, Dr., Ina-Seidel-Weg 1, D-8130 Starnberg (DE)**
Erfinder: **Batz, Hans-Georg, Dr., Traubinger-Strasse 63, D-8132 Tutzing (DE)**
Erfinder: **Lenz, Helmut, Dr., Waldschmidtstrasse 7, D-8132 Tutzing (DE)**
Erfinder: **Siedel, Joachim, Dr., Bahnhofstrasse 6, D-8131 Bernried (DE)**
Erfinder: **Pautz, Brigitte, Kientalstrasse 27, D-8036 Herrsching (DE)**
Erfinder: **Albert, Winfried, Dr., Moosstrasse 10, D-8121 Pähl (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

**EP 0 073 514 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft einen Antikörper gegen Creatinin, seine Herstellung, seine Verwendung zur Bestimmung von Creatinin auf immunologischer Basis und ein hierfür geeignetes Reagenz. In der klinischen Chemie stellt die Bestimmung des Creatinins eine der wichtigsten Methoden zur Diagnostik der Nierenfunktion dar. Gegenüber der Harnstoffbestimmung hat sie den entscheidenden Vorteil, daß die Konzentration des Creatinins im Serum von der Ernährungsweise, insbesondere von der Zufuhr proteinreicher Nahrung, praktisch unbeeinflußt bleibt.

Allerdings ist im Vergleich zum Harnstoff die Konzentration des Creatinins im Serum im Entscheidungsbereich (Obergrenze der Normalwerte 1,10 mg/dl bei Männern bzw. 0,90 mg/dl bei Frauen) außerordentlich gering. Deshalb müssen an die Empfindlichkeit und Spezifität eines Creatinin-Tests hohe Anforderungen gestellt werden.

Wegen der Bedeutung des Creatinin-Tests als Standarduntersuchungsmethode im klinischen Labor sollte dieser aber gleichzeitig mit möglichst geringem Arbeitsaufwand durchführbar und besonders für den Einsatz an Analysenautomaten geeignet sein.

Die bisher gebräuchlichste Bestimmungsmethode für Creatinin beruht auf der von M. Jaffé gefundenen Farbreaktion von Creatinin mit Pikrinsäure im alkalischen Medium. Hierbei wird nach saurer Enteiweißung der Probe (z. B. mit Trichloressigsäure oder mit Pikrinsäure) im Überstand nach Zugabe von Pikrinsäure und Alkalisieren eine rote Färbung entwickelt und photometrisch gemessen. Dieses an sich einfache Verfahren ist jedoch mit einer Reihe wesentlicher Nachteile behaftet.

Es hat sich gezeigt, daß die Jaffé-Reaktion von über 50 ebenfalls chromogenen Substanzen beeinflußt wird [Literatur: Clin. Chem. 26, 1119—1126 (1980)], insbesondere von natürlicherweise im Serum vorkommenden Bestandteilen wie Glucose, Pyruvat, Acetoacetat und Aceton und somit nicht für Creatinin spezifisch ist. Diese »Nichtcreatinin Chromogene« stören vor allem bei niedrigen Creatininkonzentrationen ($\leq$ 1 mg/dl) was zu einer Limitierung der unteren Erfassungsgrenze für Creatinin führt (»creatininblinder« Bereich).

Auch führen schon geringe Verschiebungen des pH-Wertes im Reaktionsmilieu zu einer Veränderung der Farbtiefe. Schließlich stellt auch die Verwendung teilweise ätzender und giftiger Reagentien eine Gefahrenquelle bei der Handhabung dar. Eine Reihe von Modifikationen der Jaffé-Reaktion verbessert zwar die Präzision und Durchführbarkeit, ohne jedoch diese prinzipiellen Mängel vollständig zu beheben.

Eine weitere, bekannte Methode wandelte Creatinin unter Zusatz von o-Nitrobenzaldehyd in Methylguanidin um, welches dann nach der Sakaguchi-Reaktion bestimmt wird. Bekannt ist auch eine Farbreaktion zwischen Creatinin und Kalium-Quecksilber-Thiocyanat. Beide Methoden erwiesen sich jedoch für das klinische Labor als ungeeignet.

Bekannt ist ferner, durch Kombination der Jaffé-Reaktion mit enzymatischen Teilschritten Störungen durch unspezifische Chromogene zu umgehen. Dabei wird die mit der Serumprobe vor und nach Behandlung mit Creatininamidohydrolase/Creatinkinase/ATP erhaltene Färbung in der Jaffé-Reaktion bestimmt und aus der Differenz der Extinktionen der Creatinin-Gehalt berechnet (Arch. Pharm. 3, 893—896 (1980). Diese Methode ist zwar spezifisch, die Durchführung jedoch sehr umständlich und nur schwer automatisierbar.

Ferner sind Verfahren zur Creatininbestimmung bekannt, in denen der aus Creatinin durch Einwirkung der Creatinin-Iminohydrolase freigesetzte Ammoniak bestimmt wird, entweder mit ammoniakselektiven Elektroden (Anal. Chem. 46, 246—249 (1976)) oder fluorimetrisch über den NADH-Verbrauch in der nachgeschalteten Glutamatdehydrogenase-Reaktion (Clin. Chim. Acta 100, 21—23 (1980)). Wegen der möglichen Gegenwart vergleichsweise großer Mengen an freiem Ammoniak im Probenmaterial erscheint es jedoch fraglich, ob derartige Messungen hinreichend störungsfrei gestaltet werden und damit Eingang in die Routinediagnostik finden können.

In neuester Zeit wurde ein vollenzymatischer Creatinintest beschrieben, bei dem Creatinin in Creatin umgewandelt, letzteres mit ATP zu Creatinphosphat umgesetzt und das dabei entstehende ADP in einer gekoppelten Reaktion mit Pyruvatkinase und Lactatdehydrogenase (LDH) photometrisch über die Abnahme des NADH-Gehaltes in der Reaktionslösung gemessen wird [Scand. J. clin. Lab. Invest., suppl. 29, p. 126 (1972)].

Diese Methode erfordert keine Enteiweißung der Serumprobe und ist spezifisch für Creatinin. Wegen des relativ geringen Meßsignals, selbst bei Einsatz größerer Probevolumina, ist jedoch die Empfindlichkeit des Tests im niedrigen Creatinin-Konzentrationsbereich limitiert; zudem ist durch die Notwendigkeit einer Probenleerwertbestimmung eine Anwendung der Methode in Analysenautomaten sehr erschwert.

Es besteht daher der Bedarf an einem einfachen und automatisierbaren, gleichzeitig sehr spezifischen und aus den o. g. Gründen ganz besonders im Konzentrationsbereich $\leq$ 1 mg/dl Creatinin (»creatininblinder« Bereich) sehr empfindlichen Test für Creatinin.

Der Erfindung liegt daher die Aufgabe zugrunde, die Nachteile der bisherigen Methoden zur Bestimmung von Creatinin zu beseitigen.

Gelöst wird dieses Problem erfindungsgemäß durch einen spezifischen Antikörper gegen Creatinin.

Der erfindungsgemäße Creatinin-Antikörper ist dadurch gekennzeichnet, daß er spezifisch mit

Creatinin einen Hapten-Antikörper-Komplex bildet.

Es ist überraschend, daß ein Antikörper gegen Creatinin herstellbar ist, da Creatinin eine im Körper aller Organismen, die Antikörper bilden, weit verbreitete Substanz darstellt, die selber eine Antikörperbildung nicht auslösen kann. Auch konnte wegen der relativ geringen Molekülgröße sowie der hohen Serumkonzentration nicht erwartet werden, daß Creatinin als Konjugat mit einer Haptenträgersubstanz die Bildung von Antikörper auslösen kann, die so spezifisch sind, daß sie in nennenswertem Umfang nicht einmal mit Substanzen kreuzreagieren, denen wesentliche Strukturmerkmale mit dem Creatinin gemeinsam sind wie z. B. Creatin und Harnstoff und die sich deswegen für eine quantitative Bestimmungsmethode eignen. Hierauf dürfte auch zurückzuführen sein, daß immunologische Testmethoden zwar seit etwa 40 Jahren bekannt sind und ebenfalls seit langer Zeit ein Bedarf an einer besonders im niedrigen Konzentrationsbereich verbesserten Creatininbestimmungsmethode bestanden hat, ohne daß es bisher gelungen wäre, eine derartige wirklich spezifische immunologische Creatinin-Bestimmungsmethode aufzufinden.

Die Herstellung des erfindungsgemäßen Antikörpers ist dadurch gekennzeichnet, daß man ein Konjugat von Creatinin und einem zur Antiserumbildung geeigneten Material, wie z. B. einem Protein, das über eine aliphatische oder araliphatische Carbonsäure als Brückenglied verbunden ist, als Immunogen zur Antiserumbildung verwendet.

Das im erfindungsgemäßen Verfahren zur Immunisierung verwendete Creatinin-Konjugat wird hergestellt, indem man das Creatinin an eine aliphatische oder araliphatische Carbonsäure bindet und dann die Carboxylgruppe des von der Carbonsäure stammenden Teils mit einer Haptenträgersubstanz, welche die Antiserumbildung bewirken kann, verknüpft. Als geeignet erwiesen sich hierfür die Fettsäuren mit mindestens 3 Kohlenstoffatomen, vorzugsweise mit 4 bis 16 Kohlenstoffatomen sowie Alkylseitengruppen tragende aromatische Carbonsäuren. Besonders geeignet sind Benzoesäurederivate mit einer Alkylgruppe von 1 bis 4 Kohlenstoffatomen am aromatischen Ring. Typische Beispiele sind Methylbenzoesäure, Äthylbenzoesäure und Propylbenzoesäure. Ebenfalls geeignet sind die Oligomeren dieser araliphatischen Carbonsäuren wie z. B. Methylbenzoat-methylbenzoesäure.

Die Bindung zwischen Creatinin, das hier immunologisch als Hapten anzusehen ist, und der Carbonsäure kann nach an sich bekannten Methoden unter Verwendung aktivierter Carbonsäurederivate hergestellt werden. Bevorzugt verwendet man halogenierte Carbonsäuren, insbesondere die $\omega$-Bromcarbonsäuren in wässrig alkoholischem Medium oder im Falle der entsprechenden halogenierten Carbonsäureester in wasserfreiem Medium mit anschließender Verseifung. Die besten Ergebnisse wurden mit den $\omega$-Bromcarbonsäureestern mit anschließender Verseifung erzielt, wobei Ausbeuten bis 50% erhalten wurden. In wässrig alkoholischem Medium erfolgt die Umsetzung mit dem Creatinin bei erhöhter Temperatur, zweckmäßig am Siedepunkt. Verwendet man die Carbonsäureester, so werden als Lösungsmittel zweckmäßig polare organische Lösungsmittel wie Dimethylformamid, Formamid und Tetrahydrofuran verwendet. Die Umsetzung erfolgt zweckmäßig in Gegenwart eines Amins, beispielsweise eines Mono-, Di- oder Trialkylamins. Die Verseifung des Produktes kann unter gelindem Erwärmen in wässrig alkalischem Medium durchgeführt werden. Die Kupplung kann außer über den Stickstoff auch über die $CH_2$-Gruppe durch Diazotierung mit p-Benzoesäure-diazoniumsalz erfolgen.

Die Kupplung mit der Haptenträgersubstanz erfolgt zweckmäßig in wässrig organischem Medium in Gegenwart eines Amins und eines Chlorameisensäureesters. Als besonders geeignet erwies sich Wasser/Dioxan als Reaktionsmedium.

Das so erhaltene Creatinin-Konjugat wird als Immunogen zur Antiserumbildung verwendet. Hierzu wird es nach den dem Fachmann bekannten Methoden der ausgewählten Tierspezies appliziert. Die Applikation kann beispielsweise intradermal, intramuskulär oder subkutan erfolgen. Vorzugsweise wird das Immunogen zusammen mit Freund'schem Adjuvanz zur Verstärkung der Antiserumbildung verwendet. Beim Schaf erwies sich folgendes Immunisierungsschema als gut geeignet:

| Tag | Applikation | Immunogenmenge emulgiert mit | Freund-Adjuvanz emulgiert mit |
|---|---|---|---|
| 0 | intradermal | 1 mg | + |
| 7 | intramuskulär | 1 mg | + |
| 14 | subcutan | 1 mg | + |
| 30 | intramuskulär | 1 mg | + |
| 60 | subcutan | 1 mg | + |
| usw. | subcutan | 1 mg | + |

Das so gewonnene Antiserum, welches den erfindungsgemäßen Antikörper enthält, kann nach Dialyse, bei der u. a. auch von vorn herein an die Antikörper gebundenes Serumcreatinin entfernt wird, für die Creatininbestimmung eingesetzt werden. Vorzugsweise erfolgt jedoch eine Anreicherung bzw. Reinigung des Antikörpers. Dabei wird günstig die bis 1,8 Mol/l Ammonsulfat ausfallende Proteinfraktion isoliert und durch Dialyse von niedermolekularen Bestandteilen befreit.

Eine weitere Feinreinigung kann durch Immunosorbtion erfolgen. Hierbei wird das Antikörperpräparat über trägergebundenes Creatininkonjugat gegeben. Der Antikörper wird dabei an Creatininkonjugat adsorbiert und nach Auswaschen von Verunreinigungen mit einer Creatininlösung wieder eluiert. Durch Dialyse gegen Säure oder Salzlösung kann der gebildete Komplex wieder gespalten und reiner Antikörper isoliert werden.

Eine andere geeignete Reinigungsmethode besteht in einer Dialyse der 1,8 mMol/l Ammonsulfatfraktion gegen Kochsalz, Zusatz von Creatinin und anschließendes Inkubieren mit trägergebundenem Creatinin. Hierbei läuft der creatinin-spezifische Antikörperkomplex durch die Säule und kann so von Antikörpern unerwünschter Spezifität (z. B. gegen Spacer oder Haptenträgersubstanz) abgetrennt werden.

Ein weiterer Gegenstand der Erfindung ist die Verwendung des neuen Antikörpers zur Bestimmung von Creatinin. Sie beruht darauf, daß die Bindungsreaktion zwischen einem Creatinin-Konjugat und dem Antikörper, der gegen ein Creatinin-Konjugat mit entweder derselben, bevorzugt aber mit einer anderen Haptenträgersubstanz gerichtet ist, durch Creatinin in konzentrationsabhängiger Weise gehemmt wird. Die Antikörper können z. B. in Form von Antiserum bzw. der daraus gewonnenen Immunglobulinfraktion eingesetzt werden, die zuvor durch eine geeignete Vorbehandlung wie z. B. Dialyse gegen verdünnte Propion- oder Salzsäure von anhaftendem Serum-Creatinin befreit wurde. (s. R. K. Hindawi et al., J. Immunol. Methods 37, 57—70 (1980).

Das erfindungsgemäße Verfahren zur immunologischen Bestimmung von Creatinin ist daher dadurch gekennzeichnet, daß man den Creatinin-Antikörper mit einer creatininhaltigen Probelösung inkubiert, danach ein Konjugat von Creatinin mit einer Haptenträgersubstanz zusetzt, wobei eine der beiden Komponenten, Antikörper oder Konjugat, in fester oder gelöster Form, die andere Komponente in gelöster Form vorliegt, und man die Hemmung der Bindungsreaktion zwischen den Antikörpern und dem Creatinin-Konjugat mißt.

Das zur Antikörpergewinnung verwendete und das zur Bestimmung der Bindungsreaktion eingesetzte Creatinin-Konjugat können verschiedene, alternativ aber auch dieselben Haptenträgersubstanzen enthalten (z. B. Serumalbumin), wobei in letzterem Fall Kreuzreaktionen gegen die Haptenträgersubstanz im Test dadurch selektiv kaschiert werden können, daß man der Antikörperfraktion vor Bestimmung der eigentlich interessierenden Bindungsreaktion mit dem Hapten die Haptenträgersubstanz in der zur Fällung der gegen die Haptenträgersubstanz gerichteten Antikörper notwendigen Konzentration zusetzt, wobei bei der Durchführung des Tests über die Bestimmung von Trübungsbildungen (TINIA, NINIA) die zunächst durch die Kreuzreaktion der Creatinin-Konjugat-Antikörper mit der Haptenträgersubstanz entstandenen Niederschläge entfernt werden, z. B. durch Zentrifugation oder Filtration.

Die Bindungshemmung zwischen Antikörper und Creatinin-Konjugat ist um so stärker, je mehr Creatinin dem Antikörper vor dem Vermischen mit dem Creatinin-Konjugat zugesetzt wurde. Der Hemmungseffekt ist bereits in einem Konzentrationsbereich des Creatinins in der zu messenden Probelösung unter 1 mg/dl, also dem Bereich, in dem die Brauchbarkeit der bisher vorzugsweise angewendeten bekannten Creatininbestimmungsmethoden bereits sehr eingeschränkt ist, stark ausgeprägt.

In der Literatur sind zwar bereits zahlreiche Fälle beschrieben, bei denen ein Hapten selbst bei der immunologischen Bindungsreaktion zwischen einem zugehörigen Haptenkonjugat und dem dagegen gerichteten Antiserum als Inhibitor wirkt und diese Eigenschaften auch zur quantitativen Bestimmung des freien Haptens nach verschiedenen Testverfahren (z. B. RIA, EMIT®, ELISA, TINIA oder NINIA) herangezogen wird. Bisher wurde in der Literatur jedoch nur die Gewinnung von Antikörpern gegen niedermolekulare Substanzen wie Hormone beschrieben, die im Körper des immunisierten Tieres nur in sehr geringer Menge vorkommen [$10^{-2}$—$10^{-12}$ mol/l; vgl. auch R. K. Hindawi et al., J. Immunolog. Methods 37, 57—70 (1980), Anlage].

Als Haptenträgersubstanzen eignen sich die in der Immunologie hierfür bekannten Stoffe. Beispiele hierfür sind im Prinzip alle artfremden Proteine, die im Wirtstier, welches zur Antikörperbildung herangezogen wird, nicht vorkommen, beispielsweise Serumalbumine verschiedlichen Ursprungs, sowie Key-hole-Lymphocyanin, (Lipo)-Polysaccaride, Agarose, Polylysin, Aktivkohle. Beispiele für weitere bekannte Trägersubstanzen für Haptene sind in Handbook of Experimental Immunology, Blackwell, Scientific Publications, 3. Aufl. (1978), S. 1—11 angegeben. Dort finden sich auch geeignete bekannte Methoden zur Verknüpfung von Trägersubstanz und Hapten.

Bevorzugte Haptenträgersubstanzen sind Serumalbumine verschiedener Herkunft, beispielsweise Rinderserumalbumin, Humanserumalbumin und Edestin.

Unter dem Begriff Antikörper werden im Rahmen der vorliegenden Erfindung sowohl gereinigte Antikörper als auch Antiseren, aus letzteren gewonnene Immunoglobulinfraktionen sowie Antikörperfragmente, wie $F(ab)_2$—, Fab- und Fv-Fragmente verstanden.

Für die Antikörperbildung können generell alle antikörperbildenden Lebewesen verwendet werden. Bevorzugt werden Schafe verwendet.

Die Hemmungswirkung des Creatinins auf die Bindungsreaktion zwischen dem Creatinin-Konjugat und den Antikörpern kann nach an sich bekannten immunologischen Methoden direkt gemessen werden. Als Beispiele seien erwähnt die Methoden unter Verwendung markierter Antikörper bzw. Antigene, wie RIA oder EIA, letzteres mit den Ausführungsformen ELISA oder EMIT, die Trübungsmessung der Hemmung der Immunpräzipitation zwischen Konjugat und Antikörper (TINIA-Prinzip) bzw. die entsprechende nephelometrische Methode (NINIA-Prinzip). Ferner sind anwendbar Agglutiniations-Inhibitions-Tests (PACIA = Particel Counting Immuno-Assay) und Komplementbindungsreaktionen. Diese Methoden sind dem Fachmann sämtlich bekannt und brauchen hier nicht näher beschrieben zu werden. Lediglich beispielsweise wird für Ausführungsformen der EIA auf Clin. Chim. Acta, 81 (1977), S. 1—36 und J. Clin. Chem. Biochem. 18 (1980), S. 197—208 verwiesen.

Im Falle der Enzymmarkierung können die hierfür erfolgreich verwendeten Enzyme, wie ß-Galactosidase, Peroxidase, alkalische Phosphatase, Glucoseoxidase, Glucose-6-phosphat-dehydrogenase und Luciferase erwähnt werden. Für eine Markierung mit Coenzymen kommen z. B. NAD oder NADP bzw. deren reduzierte Stufen in Betracht. Markiert kann sowohl Antikörper als auch Hapten sein.

Eine bevorzugte Methode unter den oben genannten Methoden ist die Trübungsmessung. Sie besteht darin, daß man die Hemmung der Bindungsreaktion durch Messung der Immunpräzipitation in einem vorgegebenen Zeitintervall bestimmt, wobei sowohl die nephelometrische Meßmethode (Streulichtmessung) als auch die turbidimetrische Meßmethode (Durchlichtmessung) anwendbar sind.

Eine weitere bevorzugte Methode besteht darin, daß man die Hemmung der Bindungsreaktion durch Rücktitration von nichtgebundenem Haptenträgersubstanz-Creatinin-Konjugat mit markierten Antikörpern, besonders bevorzugt enzymmarkierten Antikörpern, und Messung des gebundenen oder des nichtgebundenen Anteils der markierten Substanz bestimmt. Hierfür eignet sich z. B. die Markierung mit radioaktiven Substanzen (RIA), Enzymen bzw. Coenzymen (EIA), Fluoreszenz (FIA), Spinmarkierung (vgl. Nature New Biology, Vol. 236 (1972), S. 93/94. Als markierte Antikörper können auch markierte Anti-Antikörper verwendet werden (Doppelantikörpermethode).

Ein weiterer Gegenstand der Erfindung ist ein Reagenz zur immunologischen Bestimmung von Creatinin, welches dadurch gekennzeichnet ist, daß es Creatinin-Antikörper, ein Konjugat von Creatinin mit einer Haptenträgersubstanz und Puffersubstanz enthält.

Das erfindungsgemäße Reagenz enthält vorzugsweise auch eine Substanz, welche die Immunpräzipitation fördert. Besonders geeignet ist hierfür Polyäthylenglykol, allein oder gegebenenfalls zusammen mit einer oberflächenaktiven Substanz. Als Polyäthylenglykol eignet sich ein solches mit einem Molekulargewicht zwischen 200 und 20 000, bevorzugt wird ein Molekulargewicht von 6000 $\pm$ 2000. Geeignete Konzentrationen für Polyäthylenglykol im Reagenz liegen zwischen 0,1 und 8 Vol.-%.

Hinsichtlich des Antikörperbestandteils und der angewandten Markierung gelten die obigen Ausführungen für das erfindungsgemäße Reagenz entsprechend.

Für die turbidimetrische Bestimmungsmethode wird erfindungsgemäß ein Reagenz bevorzugt, welches 0,5 bis 500 $\mu$g/ml Konjugat aus Creatinin und Humanserumalbumin im molaren Verhältnis Creatinin : Humanserumalbumin von 2 bis 60, Antikörper gegen Creatinin im Molverhältnis 0,1 bis 10, bezogen auf das Konjugat, 0,1 bis 8 Gew.-% Polyäthylenglykol, Puffersubstanz der Ionenstärke 0,03 bis 0,4 pH 4 bis 10, enthält.

Für die EMIT-Methode enthält das erfindungsgemäße Reagenz vorzugsweise $10^{-4}$ bis $10^{-14}$ Mol/l Antikörper gegen Creatinin, bezogen auf aktive Rezeptorstellen, $10^{-4}$ bis $10^{-14}$ Mol/l Creatinin-Malatdehydrogenase-Konjugat, 0,05 bis 50 mMol/l Oxalessigsäure, 5 bis 200 mMol Phosphatpuffer, ph 6 bis 8,5 und 0,05 bis 0,4 mMol/l NADH.

Als Puffersubstanzen können im Rahmen der Erfindung die bekannten, im pH-Bereich von 4 bis 10, vorzugsweise 6 bis 9 wirksamen, verwendet werden. Die Pufferkonzentration im gelösten Reagenz sollte zwischen 0,005 und 1,0 Mol/l, vorzugsweise 0,01 bis 0,1 Mol/l, liegen. Besonders bevorzugt wird der Bereich von 0,03 bis 0,07 Mol/l.

Die Antikörperkonzentration im Test sollte zweckmäßig zwischen etwa $10^{-4}$ und $10^{-14}$ Mol/l, vorzugsweise zwischen $10^{-6}$ und $10^{-12}$ Mol/l liegen, wobei hier unter Mol/l aktive Rezeptorstellen verstanden werden.

Zur Durchführung des Verfahrens der Erfindung kann die zu analysierende Lösung unmittelbar dem Reagenz zugesetzt werden. Liegt das Creatinin in hohen Konzentrationen vor, wird die Probe zweckmäßig vorher mit Wasser verdünnt.

Die Bestimmung kann im allgemeinen bei Temperaturen zwischen 10 und 50°C, insbesondere zwischen 15 und 40°C durchgeführt werden.

Die Herstellung der Creatinin-Konjugate kann nach bekannten Methoden erfolgen, beispielsweise nach der in J. Biol. Chem. 228 (1957), S. 713—727 beschriebenen Arbeitsweise der gemischten Anhydride. Gleiches gilt für die Markierung mit einem Enzym.

Die folgenden Beispiele erläutern die Erfindung weiter. Die darin verwendeten Abkürzungen haben die nachstehend angegebene Bedeutung:

| | |
|---|---|
| CK | Creatinkinase |
| RSA | Rinderserumalbumin |
| HSA | Humanserumalbumin |
| RIA | Radio Immuno Assay |
| EMIT | Enzyme Multiplied Immunoassay Technique |
| ELISA | Enzyme Linked Immun Sorbent Assay |
| TINIA | Turbidity Inhibition Immuno Assay |
| NINIA | Nephelometric Inhibition Immuno Assay |
| BP | Beschichtungspuffer |
| IP | Inkubationspuffer |
| Tween 20® | Polyoxyäthylen-sorbitanmonolaurat |
| NAD | Nicotinamidadenin-dinucleotid |
| NADP | Nicotinamidadenin-dinucleotidphosphat |
| DMF | Dimethylformamid |
| WP | Waschpuffer |
| SP | Substratpuffer |
| PNPP-Na | p-Nitrophenylphosphat-Na |
| RT | Raumtemperatur |
| AP | Alkalische Phosphatase |
| AP-TC | Alkalische Phosphatase-Test-Kombination |

TINAQUANT-FN-Puffer:

| | |
|---|---|
| Na,K-Phosphat | 66 mMol/l pH 8,0 |
| EDTA | 10 mMol/l |
| Brij®—35 | 0,4% |
| $NaN_3$ | 0,1% |
| Polyäthylenglykol 6000 | 2,4% |
| Brij®—35 Polyäthylenglykollauryläther | 0,4% |
| RSA | 0,5% |
| HSA | 0,5% |

## A) Herstellung des Immunogens

### 1. p-(N-creatinyl)-methyl-benzoesäure

11,3 g (100 mMol) Creatinin und 21,5 g (100 mMol) p-Brom-methyl-benzoesäure werden in 250 ml Wasser-Äthanol-Mischung (1 : 1) eine Stunde am Rückfluß gekocht. Nach dem Abkühlen fällt nicht umgesetzte Brommethylbenzoesäure aus. Sie wird abfiltriert, das Filtrat wird bis auf etwa 100 ml eingeengt und dann auf eine Ionenaustauschersäule gegeben (Dowex® (Fa. SERVA) 148; 200—400 mesh, Gegenion Formiat). Bei Elution mit Wasser wird das gewünschte Produkt in Reinsubstanz gewonnen.

Fp: > 250° C, Ausbeute 2,5 g (9,5%).

$R_f$-Wert (Butanol/Eisessig/Wasser 60/15/25) = 0,43 mit einem $R_f$-Wert von 0,48 Wert als weiteres Creatinin-derivat p-(Creatinyl)-methyl-benzoat-methyl-benzoesäure isoliert, die ebenfalls als Hapten eingesetzt werden kann.

Die NMR-Spektren der beiden Substanzen bestätigen die angenommenen Strukturen.

### 2. 3(N-Creatinyl)-buttersäure

11,3 g (100 mMol) Creatinin werden in 150 ml DMF suspendiert und auf ca. 80—100° C erwärmt. Zu dieser Lösung werden unter Rühren langsam gleichzeitig 17,4 ml (120 mMol) Brombuttersäureäthylester und 16,5 ml (120 mMol) Triäthylamin getropft. Es wird dann noch 35 Stunden gerührt, wobei die Lösung fast klar wird. Die Lösung wird filtriert und das erhaltene Filtrat eingeengt. Danach wird der Rückstand in Wasser aufgenommen und mit Aktivkohle versetzt. Nach kurzem Aufkochen wird von der A-Kohle abfiltriert und erneut eingeengt. Der Rückstand wird getrocknet und anschließend aus Isopropanol umkristallisiert.

Fp: 176—178° C; Ausbeute 10,5 g (35%).

$R_f$ = 0,75 (Propanol/Ammoniak/Wasser = 5 : 3 : 1).

Die Substanz wird dabei als Hydrobromid erhalten. Der Ester wird sofort verseift, indem er in Wasser gelöst wird. Zu dieser Lösung wird bei 40—50° C unter Konstanthaltung des pH-Wertes auf pH 10 1n Natronlauge zugetropft. Wenn kein Abfall des pH-Wertes mehr eintritt, wird mit 1n Salzsäure neutralisiert. Die Lösung wird dann zur Trockene eingeengt und mit Aceton aufgenommen. Es wird

vom Ungelösten abfiltriert und erneut eingeengt. Dann wird aus Isopropanol umkristallisiert. Die Substanz liegt als Hydrochlorid vor.

Ausbeute: 5,1 g (65%).

$R_f = 0{,}66$ (Methanol/Chloroform 1 : 1).

3. Kupplung von Creatinyl-3-buttersäure an Rinderserumalbumin

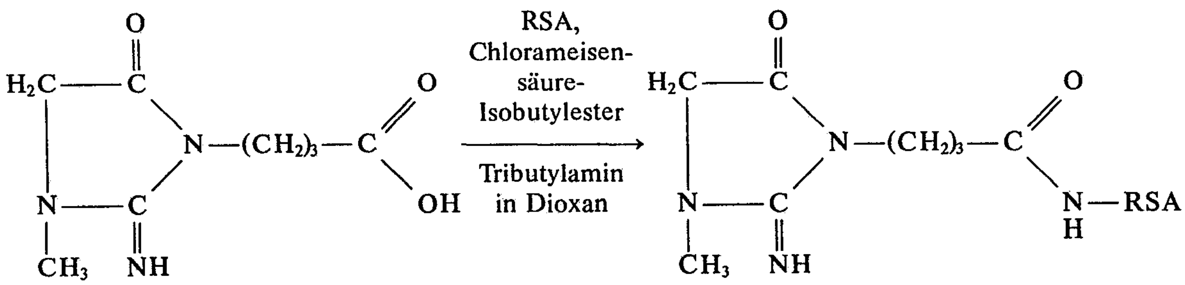


3,6 g Rinderserumalbumin werden in 250 ml Wasser/Dioxan (1 : 1) angelöst, dazu werden 5,6 ml 1n NaOH gegeben, wobei Lösung eintritt. Anschließend wird auf 4° C gekühlt. Zu dieser Lösung wird eine Lösung von 1,7 g Creatinin-buttersäure, 1,7 ml Tributylamin und 0,95 ml Chlorameisensäureisobutylester in 60 ml Dioxan und 3 ml DMF getropft.

Der Ansatz wird 24 Stunden bei 4° C gerührt, dann gegen fließendes, entsalztes Wasser 36 Stunden lang dialysiert. Danach wird die Lösung lyophilisiert.

4. Die Methode von 3. wird wiederholt, jedoch unter Verwendung der äquimolaren Menge von nach 1. erhaltener Creatinyl-methyl-benzoesäure. Man erhält so das entsprechende Konjugat mit Methylbenzoesäure als Brückenglied zwischen Creatinin und Albumin.

B) Herstellung des Antiserums

Die Immunisierung der für die Antiserumgewinnung benutzten Tiere wird wie folgt durchgeführt:

Tierspecies: Schaf
Immunogen: Creatinin-3-p-Methylbenzoesäure-RSA (Konjugat)
Immunisierungsschema:

| Tag | Applikation | Immunogen-menge emulgiert mit | Freund-Adjuvanz emulgiert mit |
|---|---|---|---|
| 0 | intradermal | 1 mg | + |
| 7 | intramuskulaer | 1 mg | + |
| 14 | subcutan | 1 mg | + |
| 30 | intramuskulaer | 1 mg | + |
| 60 | subcutan | 1 mg | + |
| usw. | subcutan | 1 mg | + |

1. Probeblutung: Tag 45

C) Aufbereitung des Antiserums

Schaf-Antiserum bei Raumtemperatur mit 1% Aerosil® versetzen, ca. 2 h rühren, abzentrifugieren, Niederschlag verwerfen.

Zum Überstand langsam festes Ammonsulfat zusetzen bis auf 1,8 mol/l und mehrere Stunden bei 4°C rühren. Mischung abzentrifugieren, Überstand verwerfen. Niederschlag in 75% des Ausgangsvolumens aufnehmen (50 mmol K-$PO_4$ und 100 mMol/l NaCl mit 0,05% $NaN_3$ pH 7,0), dann ca. 24 h gegen 0,15 Mol/l NaCl dialysieren, anschließend ggf. abzentrifugieren.

Die Lösung kann wie folgt weiterbehandelt werden:

a) Dialyse gegen 3 mMol/l HCl, ca. 8 bis 10 h, dazwischen ein Wechsel der Dialysierlösung (1 : 100-Volumenverhältnis), Niederschlag abzentrifugieren und verwerfen.
   Dialyse der Lösung gegen 10 mMol/l $PO_4$ und 150 mMol/l NaCl $<$ 12 h, (Volumenverhältnis 1 : 100). Niederschlag abzentrifugieren.

Alternative:

b) Dialyse gegen 1 ml Propionsäure/l, Dauer $>$ 2 h, dann gegen 1 mMol/l HCl (Volumenverhältnis 1 : 100), Dauer $>$ 2 h, Neutralisation mit $NaHCO_3$.
   Niederschlag abzentrifugieren und verwerfen.

Beispiel 1

TINIA-Prinzip

a) Reagentien

Es wird das Konjugat der Creatinin [3-p-methylbenzoesäure], hergestellt gemäß Beispiel A) mit Edestin und mit Rinderserumalbumin verwendet.

Das gemäß Beispiel B) gewonnene Antiserum wird gegen 1 Mol/l Propionsäure, dann gegen 1 mMol/l HCl dialysiert, dann mit Bicarbonat neutralisiert. Alternativ kann gegen 3 mmol HCl/l, dann gegen ein Gemisch von 10 mMol/l Phosphatpuffer und 150 mMol/l NaCl (pH = 7) dialysiert werden. Der Niederschlag wird abzentrifugiert.

Die Antiserumverdünnung erfolgt mit TINAQUANT®-FN-Puffer (nach 15 min nach Verdünnung wird der Niederschlag ggf. nochmals abzentrifugiert).

Creatininlösungen:
Konzentrationen von 0,2 bis 100 mg/dl TINAQUANT®-FN-Puffer.

b) Testansatz

In Kuevetten (d = 1 cm) werden zu je 1 ml Anti-RSA-Creatinin-Antiserum bzw. Anti-Edestin-Creatinin-Antiserum vom Schaf (1 : 5 bis 1 : 50 mit TINAQUANT®-FN-Puffer verdünnt) 100 µl der Creatininlösung verschiedener Konzentrationen bzw. 100 µl Puffer (für den Leerwert) hinzupipettiert und 5 bis 15 Minuten bei 25°C inkubiert.

Dann werden jeweils 20 µl einer Lösung von 1 bis 3 mg HSA- oder RSA-Creatinin-Konjugat/ml TINAQUANT®-Puffer hinzugemischt und die Zunahme der Trübung photometrisch bei 336 nm nach Zugabe des HSA-Creatinin- bzw. RSA-Creatinin-Konjugats gemessen ($E_1$ vor Start, $E_2$ 10 min nach Start). Figur 1 der Zeichnung zeigt eine so ermittelte Eichkurve, in der die Extinktionsdifferenz gegen die Creatininkonzentration aufgetragen ist.

## Beispiel 2

ELISA-Prinzip (analog dem Verfahren von J. of Immunology, 123 (1979), 1548—1550)

### a) Verwendete Reagenzien

1. BP Beschichtungspuffer
   0,2 Mol $Na_2CO_3$/l, pH 9,3 bis 9,5

2. IP Inkubationspuffer
   0,05 mol Phosphat, pH 7,2 (28,5 ml $KH_2PO_4$/71,5 ml $K_2HPO_4$)
   0,1 mol NaCl/l, 5,85 g/l
   1% Glycin
   0,05% Tween 20
   0,02% $NaN_3$

3. WP Waschpuffer
   0,15 mol NaCl/l, 8,76 g/l
   0,05% Tween 20
   0,02% $NaN_3$

4. SP Substratpuffer
   AP-Test
   1 Tabl./76 ml Puffer oder
   17 mg PNPP-$Na_2$/10 ml Puffer aus 123 854

### b) Testansatz

Mikrotiterplatten werden mit 200 µl Creatinin-HSA-Konjugat (50 µl/ml HSA/Leerwert 200 µl HSA (50 µl/ml) beschichtet bei Raumtemperatur ca. 16 h inkubiert und dann ausgesaugt. Dann werden 300 µl IP zugegeben, inkubiert, nochmals ausgesaugt, danach 300 µl WP zugegeben und erneut ausgesaugt. Anschließend erfolgt die Beladung mit Creatinin-Antiserum. Hierzu werden 1000 µl Antiserum 1 : 200 bis 1 : 2000 in IP verdünnt, mit 10 µl Creatinin-Probe bzw. 10 µl IP (für den Leerwert) 30 Minuten bei Raumtemperatur inkubiert, dann 200 µl der so erhaltenen Antiserumverdünnung auf die beschichteten Mikrotiterplatten gegeben, 60 Minuten verschlossen (Plastikbeutel), stehengelassen, ausgesaugt, 300 µl WP zugegeben und erneut ausgesaugt.

Zur Konjugatbeschichtung werden nunmehr 200 µl Kaninchen-Anti-Schaf-IgG-AP enthaltend 150 mU AP/ml (50 µl mit AP markiertem Antiantikörper (pro) 15 ml IP auf die behandelten Mikrotiterplatten gegeben, für 2 bis 3 h bei 37° C gehalten, dann ausgesaugt und mit 300 µl WP zweimal gewaschen. Zur Farbentwicklung werden 200 µl Substratpuffer (17 mg PNPP/10 ml Puffer) zugesetzt und die Mischung 30 bis 60 Minuten inkubiert. Die Auswertung erfolgt, indem man 150 µl Testlösung mit 500 µl NaOH, 0,1 mol/l bei 405 nm gegen Leerwert mißt. Fig. 2 der Zeichnung zeigt eine mit verschiedenen Creatinin-Konzentrationen erhaltene Eichkurve.

## Beispiel 3

Zu 2,00 ml 50 mM Phosphatpuffer (pH 7,5) werden nacheinander hinzugemischt:

0,002 ml Probe (oder $H_2O$ für den Leerwert)
1,00 ml $7 \times 10^{-3}$ Mol/l Oxalessigsäure in Pufferlösung (50 mMol/l Phosphat, pH 7,5)
0,01 ml $4 \times 10^{-7}$ Mol/l CREATININ-RSA-Antiserum oder $\gamma$-Globulin (Bindungsstellenkonzentration)
0,05 ml $1 \times 10^{-8}$ Mol/l CREATININ-Malat-dehydrogenase-Konjugat in Pufferlösung
0,040 ml $1,4 \times 10^{-2}$ Mol/l NADH in Wasser.

Nach Zugabe der NADH-Lösung wird die Enzymaktivität ($\Delta$E/min) spektrophotometrisch bei 340 nm und 30° C gemessen.

## Beispiel 4

Es wird, wie in Beispiel 3 beschrieben, vorgegangen, jedoch wird anstelle der Oxalessigsäure Glucose-6-phosphat in gleicher Menge und anstelle des CREATININ-MDH-Konjugats ein Konjugat aus Glucose-6-phosphat-dehydrogenase und CREATININ eingesetzt sowie statt der NADH—Lösung eine $NAD^+$-Lösung.

**Patentansprüche**

1. Creatinin-Antikörper, dadurch gekennzeichnet, daß er spezifisch mit Creatinin einen Hapten-Antikörper-Komplex bildet.

2. Verfahren zur Herstellung des Creatinin-Antikörpers nach Anspruch 1, dadurch gekennzeichnet, daß man ein Konjugat von Creatinin und einem zur Antiserumbildung geeigneten Protein, die über eine aliphatische oder araliphatische Carbonsäure als Brückenglied verbunden sind, als Immunogen zur Antiserumbildung verwendet.

3. Verfahren nach Anspruch 2, dadurch gekennzeichnet, daß man ein Konjugat verwendet, bei dem die Carbonsäurebrücke mindestens 3 Kohlenstoffatome enthält.

4. Verfahren nach Anspruch 3, dadurch gekennzeichnet, daß das Konjugat als Brückenglied eine Alkyl($C_1-C_4$)-Phenyl-Carbonsäure oder ein Oligomeres davon enthält.

5. Verfahren nach einem der Ansprüche 2 bis 4, dadurch gekennzeichnet, daß das Konjugat als Protein Serumalbumin oder Edestin enthält.

6. Verfahren nach einem der Ansprüche 2 bis 5, dadurch gekennzeichnet, daß man das Konjugat zusammen mit Freund'schem Adjuvans zur Antiserumbildung appliziert.

7. Verfahren nach einem der Ansprüche 2 bis 6, dadurch gekennzeichnet, daß man zur Reinigung aus dem gebildeten Antiserum die bis 1,8 mMol/l Ammoniumsulfat ausfallende Eiweißfraktion isoliert, dialysiert und gegebenenfalls durch Immunosorbtion an trägergebundenem Creatininkonjugat und Elution mit ungebundenem Creatinin einer Feinreinigung unterwirft.

8. Verwendung des Antikörpers von Anspruch 1 zur immunologischen Bestimmung von Creatinin, dadurch gekennzeichnet, daß man den Antikörper mit einer creatininhaltigen Probelösung inkubiert, mit einem Konjugat vom Creatinin mit einer Haptenträgersubstanz, umsetzt, wobei eine der Komponenten Antikörper und Konjugat in fester Phase oder gelöster Form, die andere Komponente in gelöster Form vorliegt, und die Hemmung der Bindungsreaktion zwischen dem Antikörper und dem Creatinin-Konjugat mißt.

9. Verwendung nach Anspruch 8, dadurch gekennzeichnet, daß man den Antikörper in Form eines Antiserums, einer daraus gewonnenen Immunglobulinfraktion oder als Antikörperfragment einsetzt.

10. Verwendung nach Anspruch 8 oder 9, dadurch gekennzeichnet, daß man die Hemmung der Bindungsreaktion durch Trübungsmessung der Immunpräzipitation in einem vorgegebenen Zeitintervall bestimmt.

11. Verwendung nach Anspruch 8 oder 9, dadurch gekennzeichnet, daß man die Hemmung der Bindungsreaktion durch Rücktitration von nichtgebundenem Haptenträgersubstanz-Creatinin-Konjugat mit markierten Antikörpern und Messung des gebundenen oder des nichtgebundenen Anteils der markierten Substanz bestimmt.

12. Verwendung nach Anspruch 11, dadurch gekennzeichnet, daß man enzym- oder coenzymmarkierte Antikörper verwendet.

13. Verwendung nach Anspruch 11, dadurch gekennzeichnet, daß man radioaktiv- oder fluoreszenzmarkierte Antikörper verwendet.

14. Verwendung nach einem der Ansprüche 8 bis 13, dadurch gekennzeichnet, daß man Creatinin-Konjugat verwendet, dessen Haptenträgersubstanz mit der zur Gewinnung des Antikörpers als Haptenträgersubstanz verwendeten Materials nicht wesentlich kreuzreagiert.

15. Verwendung nach Anspruch 11, dadurch gekennzeichnet, daß man ein Creatinin-Konjugat verwendet, welches eine Haptenträgersubstanz enthält, die mit der zur Antikörpergewinnung verwendeten Haptenträgersubstanz kreuzreagiert, freie Haptenträgersubstanz mit der Antikörperlösung mischt, den dabei gebildeten Niederschlag abtrennt und den erhaltenen Überstand als Antikörperlösung in den Test einsetzt.

16. Verwendung nach einem der Ansprüche 8 bis 15, dadurch gekennzeichnet, daß man Schafantikörper verwendet.

17. Reagenz zur immunologischen Bestimmung von Creatinin, dadurch gekennzeichnet, daß es Antikörper gegen Creatinin gemäß Anspruch 1, ein Konjugat von Creatinin mit einer Haptenträgersubstanz und Puffersubstanz enthält.

18. Reagenz nach Anspruch 17, dadurch gekennzeichnet, daß es Polyäthylenglykol enthält.

19. Reagenz nach Anspruch 17 oder 18, dadurch gekennzeichnet, daß die Antikörper als Antiserum, Immunglobulinfraktion oder Antikörperfragmente vorliegen.

20. Reagenz nach einem der Ansprüche 17 bis 19, dadurch gekennzeichnet, daß die Antikörper markiert sind.

21. Reagenz nach einem der Ansprüche 17 bis 19, dadurch gekennzeichnet, daß es 0,5 bis 500 µg/ml Konjugat aus Creatinin und Serumalbumin im molaren Verhältnis Creatinin: Serumalbumin von 1 : 1 bis 1 : 30, Antikörper gegen Creatinin im Molverhältnis 0,1 bis 10, bezogen auf das Konjugat, 0,1 bis 8 Gew.-% Polyethylenglykol und Puffersubstanz der Ionenstärke 0,03 bis 0,4 pH 4 bis 10 enthält.

22. Reagenz nach einem der Ansprüche 17 bis 20, dadurch gekennzeichnet, daß es $10^{-4}$ bis $10^{-14}$ Mol/l Antikörper gegen Creatinin-Haptenträgerkonjugat, bezogen auf aktive Rezeptorstellen, $10^{-4}$ bis $10^{-14}$ Mol/l Creatinin-Malatdehydrogenase-Konjugat, 0,05 bis 50 mMol/l Oxalessigsäure, 5 bis 200 mMol/l Phosphatpuffer, pH 6 bis 8,5, 0,05 bis 0,4 mMol/l NADH enthält.

## Claims

1. Creatinine antibody, chracterised in that it forms a hapten-antibody complex specifically with creatinine.

2. Process for the preparation of the creatinine-antibody according to claim 1, characterised in that one uses a conjugate of creatinine and a protein suitable for the antiserum formation, which are connected via an aliphatic or araliphatic carboxylic acid as bridge member, as immunogen for the antiserum formation.

3. Process according to claim 2, characterised in that one uses a conjugate in which the carboxylic acid bridge contains at least 3 carbon atoms.

4. Process according to claim 4, characterised in that the conjugate contains an alkyl-($C_1-C_4$)-phenyl-carboxylic acid or an oligomer thereof as bridge member.

5. Process according to one of claims 2 to 4, characterised in that the conjugate contains serum albumin or edestin as protein.

6. Process according to one of claims 2 to 5, characterised in that one administers the conjugate, together with Freund's adjuvant, for the antiserum formation.

7. Process according to one of claims 2 to 6, characterised in that, for the purification, one isolates from the antiserum formed the protein fraction precipitated with up to 1.8 mol/l ammonium sulphate, dialyses and optionally subjects to a high purification by immuno-sorbtion on carrier-bound creatinine conjugate and elution with unbound creatinine.

8. Use of the antibody of claim 1 for the immunological determination of creatinine, characterised in that one incubates the antibody with a creatinine-containing sample solution, reacts with a conjugats of creatinine with a hapten carrier substance, whereby one of the components, antibody and conjugate, is present in solid phase or dissolved form and the other component in dissolved form, and measures the inhibition of the binding reaction between the antibody and the creatinine conjugate.

9. Use according to claim 8, characterised in that one uses the antibody in the from of an antiserum, of an immunoglobulin fraction obtained therefrom or as antibody fragment.

10. Use according to claim 8 or 9, characterised in that one determines the inhibition of the binding reaction by turbidity measurement of the immune precipitation in a predetermined time interval.

11. Use according to claim 8 or 9, characterised in that one determines the inhibition of the binding reaction by back titration of non-bound hapten carrier substance-creatinine conjugate with marked antibodies and measurement of the bound or non-bound part of the marked substance.

12. Use according to claim 11, characterised in that one uses enzyme- or co-enzyme-antibodies.

13. Use according to claim 11, characterised in that one uses radioactive- or fluorescent-marked antibodies.

14. Use according to one of claims 8 to 13, characterised in that one uses a creatinine conjugate, the hapten carrier substance of which does not substantially cross-react with the material used for the obtaining of the antibody as hapten carrier substance.

15. Use according to claim 11, characterised in that one uses a creatinine conjugate which contains a hapten carrier substance which cross-reacts with the hapten carrier substance used for obtaining the antibody, mixes free hapten carrier substance with the antibody solution, separates off the precipitate thereby formed and uses the supernatant obtained as antibody solution in the test.

16. Use according to one of the claims 8 to 15 characterised in that one uses sheep antibody.

17. Reagent for the immunological determination of creatinine, chracterised in that it contains antibody against creatinine according to claim 1, a conjugate of creatinine with a hapten carrier substance and buffer substance.

18. Reagent according to claim 17, characterised in that it contains polyethylene glycol.

19. Reagent according to claim 17 or 18, characterised in that the antibodies are present as antiserum, immunoglobulin fraction or antibody fragments.

20. Reagent according to one of claims 17 to 19, characterised in that the antibodies are marked.

21. Reagent according to one of claims 17 to 19, characterised in that it contains 0.5 to 500 μg./ml. of conjugate of creatinine and serum albumin in the molar ratio of creatinine: serum albumin of 1 : 1 to 1 : 30, antibody against creatinine in the mole ratio of 0.1 to 10, referred to the conjugate, 0.1 to 8 wt.% of polyethylene glycol and buffer substance of the ionic strength of 0.03 to 0.4, pH 4 to 10.

22. Reagent according to one of claims 17 to 20, characterised in that it contains $10^{-4}$ to $10^{-14}$ mol/l. of antibody against creatinine-hapten carrier conjugate, referred to active receptor sites, $10^{-4}$ to $10^{-14}$ mole/l. of creatinine-malate dehydrogenase conjugate, 0.05 to 50 mmole/l. oxalacetic acid, 5 to 200 mmole/l. of phosphate buffer, pH 6 to 8.5, 0.05 to 0.4 mmole/l. of NADH.

## Revendications

1. Anticorps anti-créatinine, caractérisé en ce qu'il forme spécifiquement avec la créatinine un complexe haptène-anticorps.

2. Procédé de préparation de l'anticorps anti-créatinine suivant la revendication 1, caractérisé en ce

qu'on utilise comme immunogène pour la formation d'antisérum un conjugué de créatinine et d'une protéine appropriée à la formation d'antisérum, qui sont liés par l'intermédiaire d'un acide carboxylique aliphatique ou araliphatique comme élément de pontage.

3. Procédé suivant la revendication 2, caractérisé en ce qu'on utilise un conjugué dans lequel le pont acide carboxylique contient au moins 3 atomes de carbone.

4. Procédé suivant la revendication 3, caractérisé en ce que le conjugué contient comme élément de pontage un acide alcoyl(en $C_1-C_4$)-phényl-carboxylique ou un oligomère de celui-ci.

5. Procédé suivant l'une des revendications 2 à 4, caractérisé en ce que le conjugué contient comme protéine de la sérumalbumine ou de l'édestine.

6. Procédé suivant l'une des revendications 2 à 5, caractérisé en ce qu'on applique le conjugué ensemble avec de l'adjuvant de Freund pour la formation d'antisérum.

7. Procédé suivant l'une des revendications 2 à 6, caractérisé en ce que pour la purification, on isole de l'antisérum formé la fraction protéique précipitant jusqu'à 1,8 mol/l de sulfate d'ammonium, en ce que'on la dialyse et le cas échéant la soumet à une purification fine par immunosorption sur conjugué de créatinine lié à un support et élution avec de la créatinine non liée.

8. Utilisation de l'anticorps suivant la revendication 1 pour le dosage immunologique de la créatinine, caractérisé en ce qu'on fait incuber l'anticorps avec une solution échantillon contenant de la créatinine, ce qu'on le fait réagir avec un conjugué de la créatinine avec une substance de support d'haptène, l'un des constituants, anticorps et conjugué, étant sous forme de phase solide ou sous forme dissoute, l'autre constituant sous forme dissoute, et en ce qu'on mesure l'inhibition de la réaction de liaison entre l'anticorps et le conjugué de la créatinine.

9. Utilisation suivant la revendication 8, caractérisée en ce qu'on utilise l'anticorps sous la forme d'un antisérum, d'une fraction d'immunoglobuline obtenue à partir de celui-ci ou sous la forme de fragment d'anticorps.

10. Utilisation suivant la revendication 8 ou 9, caractérisée en ce qu'on détermine l'inhibition de la réaction de liaison par mesure de trouble de l'immunoprécipitation dans un intervalle de temps déterminé à l'avance.

11. Utilisation suivant la revendication 8 ou 9, caractérisée en ce qu'on détermine l'inhibition de la réaction de liaison par titrage en retour du conjugué substance support d'haptène-créatinine non lié avec des anticorps marqués et mesure de la fraction liée ou de la fraction non liée de la substance marquée.

12. Utilisation suivant la revendication 11, caractérisée en ce qu'on utilise des anticorps marqués par une enzyme ou une coenzyme.

13. Utilisation suivant la revendication 11, caractérisée en ce qu'on utilise des anticorps marqués par radioactivité ou par fluorescence.

14. Utilisation suivant l'une des revendications 8 à 13, caractérisée en ce qu'on utilise un conjugué de créatinine dont la substance support d'haptène ne donne pas lieu d'une manière importante à une réaction croisée avec la matière utilisée comme substance de support d'haptène pour la préparation de l'anticorps.

15. Utilisation suivant la revendication 11, caractérisée en ce qu'on utilise un conjugué de créatinine qui contient une substance de support d'haptène qui donne lieu à une réaction croisée avec la substance de support d'haptène utilisée pour la préparation de l'anticorps, en ce qu'on mélange la substance support d'haptène libre avec la solution d'anticorps, en ce qu'on sépare le précipité qui se form à cette occasion, et en ce qu'on utilise dans l'essai le liquide surnageant obtenu comme solution d'anticorps.

16. Utilisation suivant l'une des revendications 8 à 15, caractérisé en ce qu'on utilise des anticorps de mouton.

17. Réactif pour le dosage immunologique de la créatinine, caractérisé en ce qu'il contient des anticorps anti-créatinine suivant la revendication 1, un conjugué de la créatinine avec une substance support d'haptène et une substance tampon.

18. Réactif suivant la revendication 17, caractérisé en ce qu'il contient du polyéthylèneglycol.

19. Réactif suivant la revendication 17 ou 18, caractérisé en ce que les anticorps sont présents sous forme d'antisérum, de fraction d'immunoglobuline ou de fragments d'anticorps.

20. Réactif suivant l'une des revendications 17 à 19, caractérisé en ce que les anticorps sont marqués.

21. Réactif suivant l'une des revendications 17 à 19, caractérisé en ce qu'il contient 0,5 à 500 $\mu$g/ml de conjugué de créatinine et de sérumalbumine dans le rapport molaire créatinine : sérumalbumine de 1 : 1 à 1 : 30, des anticorps anti-créatinine dans le rapport molaire de 0,1 à 10, par rapport au conjugué, 0,1 à 8% en poids de polyéthylèneglycol et de la substance tampon de force ionique 0,03 à 0,4, pH 4 à 10.

22. Réactif suivant l'une des revendications 17 à 20, caractérisé en ce qu'il contient $10^{-4}$ à $10^{-14}$ mole/l d'anticorps contre le conjugué créatinine-support d'haptène, par rapport aux emplacements récepteurs actifs, $10^{-4}$ à $10^{-14}$ mole/l de conjugué créatinine-malate déshydrogénase, 0,05 à 50 mmoles/l d'acide oxalacétique, 5 à 200 mmoles/l de tampon phosphate, pH 6 à 8,5, 0,05 à 0,4 mmole/l de NADH.

FIG. 1

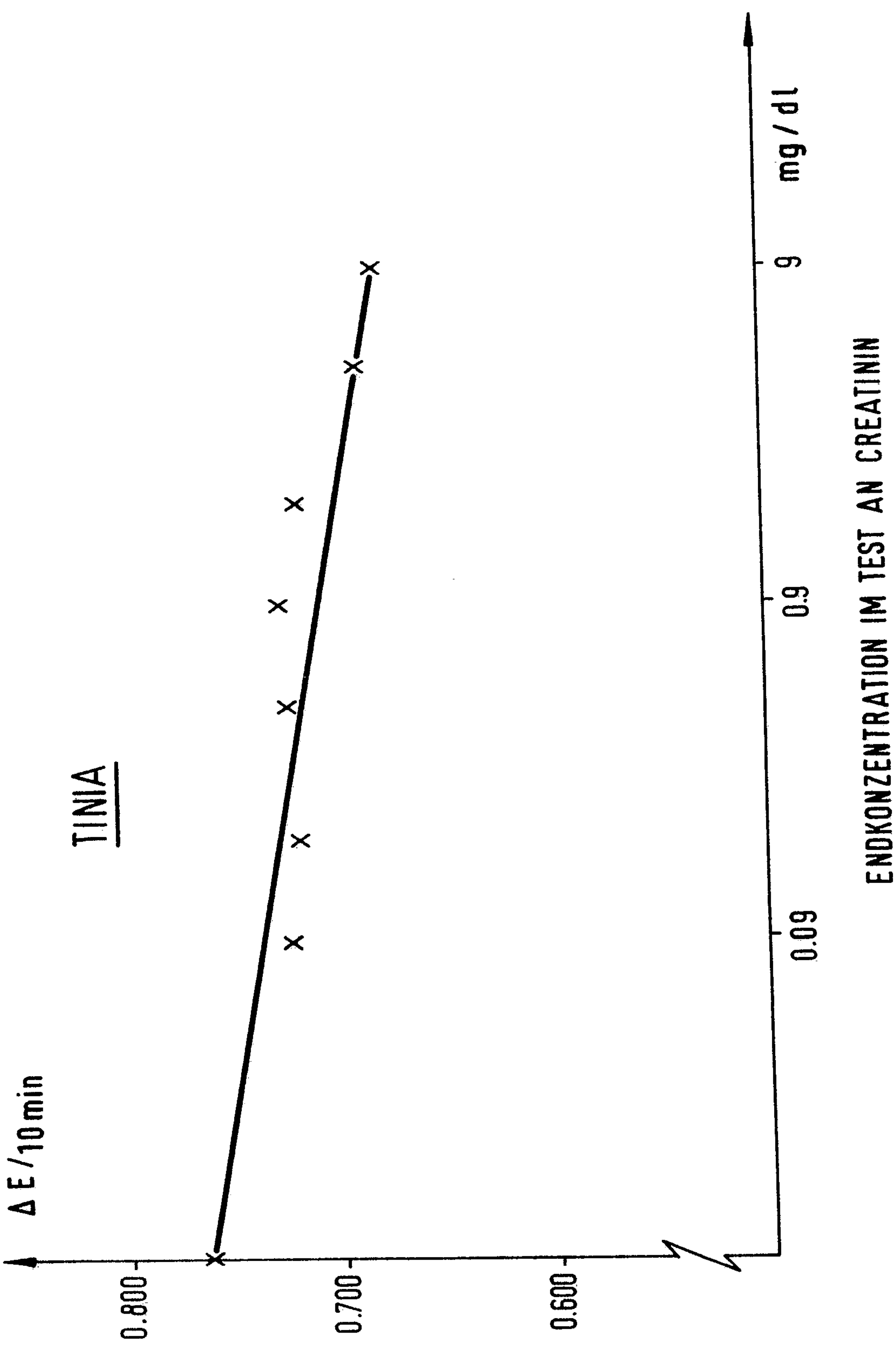
TINIA
366nm
ΔE/10min
0.800
0.700
0.600
0.09
0.9
9
mg/dl
ENDKONZENTRATION IM TEST AN CREATININ

FIG. 2

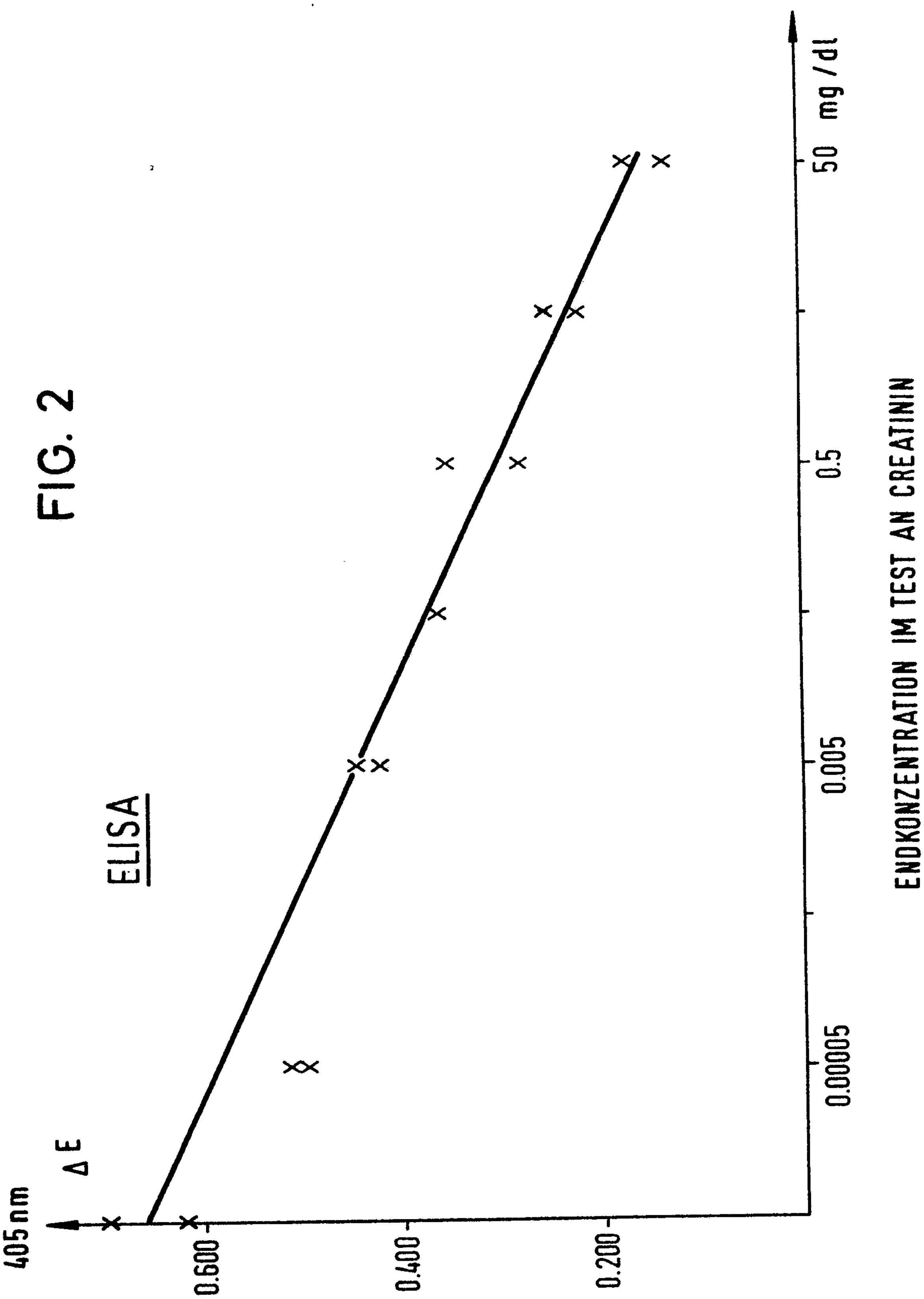
ELISA
ΔE
405 nm
0.600
0.400
0.200
0.00005
0.005
0.5
50 mg/dl
ENDKONZENTRATION IM TEST AN CREATININ